# EUROPÄISCHE PATENTSCHRIFT

(11) **EP 2 052 747 B1**
(45) Veröffentlichungstag und Bekanntmachung des Hinweises auf die Patenterteilung: **21.12.2016**
(21) Anmeldenummer: 08017156.4
(22) Anmeldetag: 30.09.2008
(51) Int. Cl.: A61L 24/00

(54) **Einkomponenten-Knochenzementpasten und Verfahren zu deren Aushärtung**
Single component bone cement pastes and method for their hardening
Pâtes de ciment osseux à composant unique et leur procédé de durcissement

(30) Priorität: 22.10.2007 DE 102007050760; 30.10.2007 DE 102007052116
(43) Veröffentlichungstag der Anmeldung: 29.04.2009
(73) Patentinhaber: Heraeus Medical GmbH, 61273 Wehrheim (DE)
(72) Erfinder: Vogt, Sebastian, Dr., 99092 Erfurt (DE); Büchner, Hubert, Dr., 64354 Reinheim (DE)
(74) Vertreter: Heraeus IP

(56) Entgegenhaltungen:
- DE-A1- 19 928 238
- US-A- 4 396 476
- US-A- 5 797 873

## Beschreibung

Gegenstand der Erfindung sind Einkomponenten-Knochenzementpasten und Verfahren zu deren Aushärtung.

PMMA-Knochenzemente sind seit Jahrzehnten bekannt und gehen auf die grundlegenden Arbeiten von Sir Charnley zurück (Charnley, J.: Anchorage of the femoral head prosthesis of the shaft of the femur. J. Bone Joint Surg. 42 (1960) 28-30.). Der Grundaufbau der PMMA-Knochenzemente ist seit dem prinzipiell gleich geblieben. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente besteht aus einem oder mehreren Polymeren, die auf Basis von Methylmethacrylat und Comonomeren, wie Styren, Methylacrylat oder ähnlichen Monomeren durch Polymerisation, vorzugsweise Suspensionspolymerisation, hergestellt sind, einem Röntgenopaker und dem Initiator Dibenzoylperoxid. Beim Vermischen der Pulverkomponente mit der Monomerkomponente entsteht durch Quellung der Polymere der Pulverkomponente im Methylmethacrylat ein plastisch verformbarer Teig. Gleichzeitig reagiert der Aktivator N,N-Dimethyl-p-toluidin mit dem Dibenzoylperoxid, dass unter Bildung von Radikalen zerfällt. Die gebildeten Radikalen initiieren die radikalische Polymerisation des Methylmethacrylates. Mit fortschreitender Polymerisation des Methylmethacrylates erhöht sich die Viskosität des Zementteigs bis der Teig erstarrt und damit ausgehärtet ist.

Grundlegende mechanische Anforderungen an PMMA-Knochenzemente, wie 4-Biegefestigkeit, Biegemodul und Druckfestigkeit, sind in der ISO 5833 beschrieben. Für den Anwender der PMMA-Knochenzemente ist die Eigenschaft der Klebfreiheit des Knochenzementes von wesentlicher Bedeutung. Der Begriff Klebfreiheit ist in der ISO5833 definiert. Die Klebfreiheit zeigt bei konventionellen PMMA-Knochenzementen an, dass der Zement nach dem Vermischen der Komponenten durch Quellung der im Zementpulver enthaltenen Polymere im Monomer die Verarbeitungsphase erreicht hat. Grundsätzlich muss ein PMMA-Knochenzement klebfrei sein, damit der Anwender den Zement formen und applizieren kann. Der PMMA-Knochenzement darf nicht an den Handschuhen und an Applikationshilfen, wie Mischsystemen, Tiegeln oder Spateln kleben.

Ein Nachteil der konventionellen PMMA-Knochenzemente für den Zementproduzenten besteht darin, dass sowohl die Pulverkomponente als auch Monomerkomponente jeweils doppelt steril verpackt hergestellt werden müssen. Das bedeutet, es sind mindestens vier sterile Packmittel für eine Knochenzementpackung notwendig.

Ein weiterer Nachteil der bisherigen PMMA-Knochenzemente für den medizinischen Anwender besteht darin, dass die flüssige Monomerkomponente mit der Pulverkomponente unmittelbar vor der Zementapplikation in einem Mischsystem oder in Tiegeln vermischt werden muss. Dabei können leicht Mischungsfehler auftreten, welche die Zementqualität negativ beeinflussen können. Nach der Vermischung der Monomerkomponente mit der Pulverkomponente muss je nach Zementtyp eine gewisse Zeit gewartet werden, bis der Zementteig klebfrei ist und appliziert werden kann. Danach hat der Anwender eine mehr oder weniger kurze Verarbeitungszeit zur Verfügung in der Totalendoprothesen positioniert werden können oder Knochenkavitäten wie bei der Kyphoplastie und Vertebroplastie aufgefüllt werden können. Während der Verarbeitungszeit verändert sich die Viskosität des Zementteigs infolge der zunehmenden Quellung der Polymerpartikel im Monomer und der fortschreitenden Polymerisation des Monomers. Die relativ kurze Verarbeitungszeit ist ein wesentlicher Nachteil der bisherigen Knochenzemente. Besonders nachteilig sind kurze Verarbeitungszeiten bei der Kyphoplastie und Vertebroplastie. Wünschenswert wäre ein Zement, insbesondere für die Vertebroplastie und Kyphoplastie, bei dem die Viskosität des Zementteigs während der Zementapplikation zeitlich konstant bleibt. Der Zement sollte sofort ohne zusätzliche Wartephase nach der Applikation gezielt ausgehärtet werden können.

US 4,396,476 beschreibt eine Dentalmasse, die härtbar ist, indem sie Wärme oder lektromagnetischer Strahlung ausgesetzt wird. Sie enthält, ein unvernetztes Polymer, ein polymerisierbares Monomer und ein vernetztes Polymer. Es können ferner übliche Pigmente oder Füllstoffe, wie Siliciumdioxid oder Titandioxid enthalten sein. Die Dentalmasse wird thermisch oder photochemisch ausgehärtet.

Die Aufgabe der Erfindung besteht deshalb darin, einen PMMA-Knochenzement zu entwickeln, der die Nachteile der bekannten PMMA-Knochenzemente mindert oder beseitigt.

Der zu entwickelnde PMMA-Knochenzement soll insbesondere in einer Form für den Anwender bereitgestellt werden, dass ein umständliches, mit vielen Fehlermöglichkeiten behaftetes Vermischen von Zementkomponenten vermieden wird. Der Knochenzement soll möglichst einfach applizierbar sein. Der Zement soll so bereitgestellt werden, dass eine Wartephase bis zur Erreichung der Klebfreiheit nicht notwendig ist. Der Zementteig muss eine solche Viskosität und Kohäsion aufweisen, dass er dem Blutungsdruck bis zur Aushärtung standhält. Weiterhin soll die Belastung der Anwender durch Monomerdämpfe weitgehend vermieden werden. Eine weitere Aufgabe besteht darin, dass der PMMA-Knochenzement durch eine äußere Einwirkung gezielt zur Aushärtung gebracht werden kann.

Die Aufgabe der Erfindung wird Einkomponenten-Knochenzementpasten nach Anspruch 1 gelöst. Vorteilhafte Ausgestaltungen ergeben sich aus den weiteren Ansprüchen.

Die Komponenten der Knochenzementpasten sind:
**AI.** mindestens ein radikalisch polymerisierbares, Methacrylatmonomer;
**AII**. mindestens ein in **AI** lösliches Polymer;
**AIII.** mindestens ein in **AI** nicht lösliches partikulären Polymer mit einer Partikelgröße kleiner 500 µm;
**AIV.** mindestens ein in **AI** löslicher, thermisch zerfallender radikalischer Initiator;
**AV.** mindestens ein elektrisch leitfähiger Röntgenopaker.

Als Methacrylatmonomere sind difunktionelle Methacrylate bevorzugt, insbesondere Ethylenglykoldimethacrylat, Butan-1,3-diol-dimethacrylat, Butan-1,4-diol-dimethacrylat und Hexan-1,6-diol-dimethacrylat. Diese Monomere polymerisieren nach der Initiierung innerhalb sehr kurzer Zeit, und sie haben bei Normaldruck Siedepunkte von über 110 °C und sind wenig flüchtig. Das Hexan-1,6-diol-dimethacrylat hat neben seinem hohen Siedepunkt noch den wesentlichen Vorteil, dass es in Wasser bei Raumtemperatur praktisch unlöslich ist. Es ist auch möglich, zusätzliche Monomere mit Haftgruppen in den PMMA-Knochenzement zu integrieren, wie z. B. Methacrylsäure-2-hydroxyethylester. Dadurch kann die Anbindung des PMMA-Knochenzementes an die Gelenkendoprothesen gezielt beeinflusst werden.

Als im Methacrylatmonomer/Methacrylatmonomeren lösliche Polymere sind Poly-methylmethacrylat-copolymere bevorzugt, besonders Poly-methylmethacrylat-co-methylacrylat und Polymethylmethacrylat-co-styren.

Weiterhin sind vernetztes Poly-methylmethacrylat und vernetztes Poly-methylmethacrylat-co-methylacrylat als im Methacrylatmonomer/Methacrylatmonomeren nicht lösliches partikuläres Polymer bevorzugt.

Thermisch zerfallende Initiatoren sind dem Fachmann bekannt. Gängige Beispiele sind Peroxide wie Dibenzoylperoxid und Dilauroylperoxid, Bevorzugt sind Azoverbindungen, davon besonders 2,2'-Azobis(isobutyronitril). Daneben ist es auch möglich, andere Azo-Initiatoren zu verwenden, die eine geringere oder auch eine höhere Zerfallstemperatur als 2,2'-Azobis(isobutyronitril) besitzen.

Bevorzugte Gewichtsanteile im pastenförmigen Einkomponenten-Knochenzement sind 2,0-20,0 Gewichtsteile elektrisch leitfähiger Röntgenopaker; 25,0-45,0 Gewichtsteile Methacrylatmonomer/Methacrylatmonomere; 2,0-35,0 Gewichtsteile lösliche Polymere; 30,0-70,0 Gewichtsteile unlösliche Polymere und 0,5-4,0 Gewichtsteile thermisch zerfallender Initiator.

Bei den elektrisch leitfähigen Röntgenopakern handelt es sich um Partikel von Kobalt, Eisen, NdFeB, SmCo, Kobalt-Chrom-Stahl, Zirkonium, Hafnium, Titan, Titan-Aluminium-Silizium-Legierungen und Titan-Niobium-Legierungen mit einer Partikelgröße von 0,5-500 µm. In dem elektrisch leitfähigen Röntgenopaker können durch magnetische Wechselfelder Wirbelströme induziert werden. Es können auch im pastenförmigen Einkomponenten-Knochenzement zusätzliche konventionelle Röntgenopaker enthalten sein, insbesondere Zirkoniumdioxid, Bariumsulfat, Tantal und biokompatible Calciumsalze.

Besonders als elektrisch leitfähige Röntgenopaker bevorzugt sind ferromagnetische Partikel.

Es können zusätzlich pharmazeutische Wirkstoffe enthalten sein, besonders aus den Gruppen der Antibiotika, Hormone, Wachstumsfaktoren und Antiphlogistika. Als Antibiotika kommen vor allem Aminoglykosid-Antibiotika, Glykopeptid-Antibiotika, Fluorchonolon-Antibiotika, Lincosamid-Antibiotika und Oxazolidinon-Antibiotika in Betracht. Bevorzugt sind dabei Gentamicin, Tobramycin, Amikacin, Teicoplanin, Vancomycin, Ramoplanin, Dalbavancin, Moxifloxaxin, Ciprofloxacin, Lincosamin, Clindamycin und Linezolid bevorzugt. Die Antibiotika können in partikulärer oder auch in gelöster Form im pastenförmigen Einkomponenten-Knochenzement vorliegen.

Zusätzlich können auch ein oder mehrere biokompatible partikuläre oder im Methacrylatmonomer/Methacrylatmonomeren lösliche Elastomere enthalten sein, besonders Polybutadien-co-styrol. Dadurch ist es möglich, besonders schlagzähe und ermüdungsfeste Zemente herzustellen.

Weiterhin kann gegebenenfalls ein elektrisch leitfähiges Additiv zur Verbesserung der Kontaktierung der Röntgenopakerpartikel enthalten sein. Als solche Additive kommen nanopartikuläre Metallpartikel, leitfähige Polymere und Graphit in Betracht.

Erfindungsgemäße Knochenzementpasten können als selbsthärtende Kunststoffe zur Fixierung von primären Totalgelenkendoprothesen und von Revisions-Gelenkendoprothesen verwendet werden, ferner als selbsthärtende Füllmaterialien für die Vertebroplastie, Kyphoplastie und zur Femurhalsaugmentation, oder auch als selbsthärtende Implantatmaterialien zur Herstellung von lokalen Wirkstofffreisetzungssystemen. So ist es z. B. möglich, mit einem Antibiotikum enthaltenden erfindungsgemäßen PMMA-Knochenzement kugelförmige oder bohnenförmige Implantate zu formen, die als lokale Wirkstofffreisetzungssysteme eingesetzt werden können.

Die PMMA-Knochenzement-Paste kann auch zur Herstellung weiterer Einkomponenten-Knochenzementen eingesetzt werden.

Die Erfindung betrifft auch ein Verfahren zur Aushärtung des pastenförmigen Einkomponenten-Knochenzementes, bei dem der pastenförmige Knochenzement einem magnetischen Wechselfeld mit einer Frequenz im Bereich von 500 Hz bis 50 kHz ausgesetzt wird. Es werden Wirbelströme im Röntgenopaker induziert, die zu einer Erwärmung des Opakers führen. Durch Wärmeübertragung wird auch der Initiator aufgeheizt und zum thermischen Zerfall gebracht. Die radikalische Polymerisation des Methacrylatmonomers/Methacrylatmonomeren setzt ein und führt zur Aushärtung des Zementes. Der besondere Vorteil der induktiven Erwärmung ist, dass sich nur elektrisch leitfähige Materialien infolge der Induktion von Wirbelströmen aufheizen können und, dass humanes Gewebe durch magnetische Wechselfelder nicht aufgeheizt wird.

Bei einem weiteren Verfahren zur Aushärtung des pastenförmigen Einkomponenten-Knochenzementes kann der pastenförmige Knochenzement durch Induktion bis zum Einsetzen des Zerfalls des Initiators erwärmt werden.

Die Erfindung wird durch nachstehende Beispiele erläutert, ohne jedoch die Erfindung einzuschränken. Teile und Prozentangabe beziehen sich wie in der übrigen Beschreibung auf das Gewicht, sofern nicht anders angegeben.

### Beispiel 1

### Pasten 1-5

Für die nachfolgend beschriebenen Pasten wurde ein partikuläres Poly-methylmethacrylat-co-methylacrylat (Molmasse ca. 800000; ca. 5-8 % Methylacrylat-Anteil, Korngröße < 63 µm) verwendet, das als Polymer 1 bezeichnet wird. Dieses Polymer ist in Hexan-1,6-diol-dimethacrylat und in Butan-1,4-diol-dimethacrylat unlöslich. Weiterhin wurde ein Poly-methylmethacrylat-co-methylacrylat (Molmasse ca. 600000; ca. 50 % Methylacrylat-Anteil) eingesetzt. Dieses Polymer ist in Hexan-1,6-diol-dimethacrylat und in Butan-1,4-diol-dimethacrylat löslich.

Es wurde zuerst jeweils das Polymer 2 in der entsprechenden Menge Hexan-1,6-diol-dimethacrylat gelöst. In diese Lösungen wurde dann das Polymer 1 durch Kneten bei Raumtemperatur eingebracht. Die Pasten waren klebfrei und streichfähig. Sie zeigten ab 48 Stunden nach der Herstellung keine Volumenveränderung mehr.

| Pasten-Komponenten | Zusammensetzung | | | | |
|---|---|---|---|---|---|
| | Paste 1 | Paste 2 | Paste 3 | Paste 4 | Paste 5 |
| Polymer 1 | 20,000 g | 20,000 g | 20,000 g | 15, 950 g | 21,312 g |
| Polymer 2 | 1,785 g | 1,190 g | 0, 833 g | 2,392 g | 1, 588 g |
| Hexan-1,6-dioldimethacrylat | 10,115 | 10,710 g | 11,067 g | 13,557 g | 9,000 |

### Beispiel 2

### Pasten 6-10

Die Herstellung erfolgte analog der Pasten 1-5 jedoch mit Butan-1,4-diol-dimethacrylat.

| Pasten-Komponenten | Zusammensetzung | | | | |
|---|---|---|---|---|---|
| | Paste 6 | Paste 7 | Paste 8 | Paste 9 | Paste 10 |
| Polymer 1 | 20,000 g | 20,000 g | 20,000 g | 15,950 g | 21,312 g |
| Polymer 2 | 1,785 g | 1,190 g | 0,833 g | 2,392 g | 1,588 g |
| Hexan-1,6-dioldimethacrylat | 10,115 | 10,710 g | 11,067 g | 13,557 g | 9,000 |

Die Pasten waren klebfrei und streichfähig. Sie zeigten ab 48 Stunden nach der Herstellung keine Volumenveränderung mehr.

### Beispiel 3

### Zweikomponenten-Pastenzement

Die Herstellung erfolgte analog der Pasten 1-10 auf Basis der Rezeptur von Paste 1. Es wurde hierbei jedoch das Initiationssystem CaCHEBA (Calciumsalz der 1-Cyclohexyl-5-ethyl-barbitursäure)/Kupfercarbonat/ALIQUAT/2-Ethyl-hexansäure verwendet. Die Pasten A und B waren klebfrei, streichfähig und visuell homogen.

| Pasten-Komponenten | Zusammensetzung | |
|---|---|---|
| | Paste A | Paste B |
| Polymer 1 | 4,998 g | 5,250 g |
| Polymer-Lösung | 3,500 g | 3,500 g |
| Zirkoniumdioxid-Kupfercarbonat-Gemisch | 1,002 g | - |
| Zirkoniumdioxid | - | 1,000 g |
| CaCHEBA | 0,500 g | |
| 2-Ethyl-hexansäure | - | 0,200 g |
| ALIQUAT 336 | - | 0,050 g |

Nach Vermischung der Komponenten A und B war die entstandene Paste ebenso problemlos formbar und streichfähig. Die Aushärtung setzte 2 Minuten und 50 Sekunden nach dem Vermischen ein.

### Beispiel 4

### Zweikomponenten-Pastenzement

| Pasten-Komponenten | Zusammensetzung | |
|---|---|---|
| | Paste A | Paste B |
| Degacryl 6690 | 4,998 g | 5,250 g |
| Polymer-Lösung | 3,500 g | 3,500 g |
| Zirkoniumdioxid-Kupfercarbonat-Gemisch | 0,501 g | - |
| Zirkoniumdioxid | 0,501 g | 1,000 g |
| CaCHEBA | 0,500 g | |
| 2-Ethyl-hexansäure | - | 0,200 g |
| ALIQUAT 336 | - | 0,050 g |

Bei Degacryl 6690 handelt es sich um ein vernetztes Polymethylmethacrylat. Nach dem Vermischen der klebfreien Komponenten A und B entstand eine klebfreie Paste. Die Aushärtung setzte 4 Minuten und 5 Sekunden nach dem Vermischen der Komponenten A und B ein.

### Beispiel 5

Für die folgenden Pasten werden ein Polymer 1 (Poly-methylmethacrylat-co-methylacrylat, Methacrylat-Anteil 2-10 %, Molmasse ca. 800 000) und ein Polymer 2 (Poly-methylmethacrylat-co-methylacrylat, Methacrylat-Anteil 40-50 %, Molmasse ca. 800 000, Partikelgröße < 63 µm) eingesetzt. Das Polymer 1 ist in Ethylenglykoldimethacrylat, Butan-1,4-diol-dimethacrylat und Hexan-1,6-diol-dimethacrylat löslich. Das Polymer 2 ist in Ethylenglykol-dimethacrylat löslich und nicht löslich in Butan-1,4-diol-dimethacrylat und in Hexan-1,6-dio-dimethacrylat. Weiterhin wurde das vernetzte Poly-methylmethacrylat Degacryl 6690 (Partikelgröße < 100 µm) eingesetzt. Das Monomer Ethylenglykoldimethacrylat (EGMA) wurde von der Firma Fluka bezogen und das Hexan-1,6-diol-dimethacrylat (HDMA) von der Firma Degussa. Als elektrisch leitfähiger Röntgenopaker wurde Eisenpulver (Fe-Pulver) und Kobaltpulver (Co-Pulver) mit einer Korngröße von ca. 100-200 µm eingesetzt. Weiterhin wurde handelsübliches 2,2'-Azobis(isobutyronitril) (AIBN) verwendet.

| | Zusammensetzung | | | | |
|---|---|---|---|---|---|
| Paste | Röntgenopaker | Monomer | lösliches Polymer | Unlösliches Polymer | Initiator |
| 11 | 1,50 g Fe-Pulver | 3,50 g (HDMA) | 0,35 g Polymer 1 | 4,45 g Polymer 2 | 0,2 g AIBN |
| 12 | 1,50 g Fe-Puler | 3,50 g (HDMA) | 0,35 g Polymer 1 | 4,35 g Polymer 2 | 0,3 g AIBN |
| 13 | 1,50 g Fe-Puler | 3,50 g (HDMA) | 0,35 g Polymer 1 | 4,25 g Polymer 2 | 0,4 g AIBN |
| 14 | 1,50 g Co-Puler | 4,0 g (EGMA) | 0,35 g Polymer 2 | 3,95 g Degacryl 6690 | 0,2 g AIBN |
| 15 | 2,00 g Co-Pulver | 3,50 g (HDMA) | 0,30 g Polymer 1 | 4,0 g Polymer 2 | 0,2 g AIBN |

Die Zemente der Beispiele 11-15 stellen pastenförmige Massen dar, die problemlos streichbar und formbar sind. Die Aushärtung erfolgte mit Hilfe einer bei konventionellen Induktionsherden entlehnten Induktionsheizung (Spule mit Steuerungselektronik, Frequenz 25 kHz). Die Polymerisation setzte nach 30-40 Sekunden ein und führte zu stabilen Formkörpern.

## Patentansprüche

1. Einkomponenten PMMA-Knochenzement-Paste enthaltend
**AI**. mindestens ein radikalisch polymerisierbares, Methacrylatmonomer,
**AII**. mindestens ein in **AI** lösliches Polymer,
**AIII.** mindestens ein in **AI** nicht lösliches partikulären Polymer mit einer Partikelgröße kleiner 500 µm,
**AI V.** mindestens einen in **AI** löslichen, thermisch zerfallenden radikalischen Initiator,
**AV.** Partikel von Kobalt, Eisen, NdFeB, SmCo, Kobalt-Chrom-Stahl, Zirkonium, Hafnium, Titan, Titan-Aluminium-Silizium-Legierungen und Titan-Niob-Legierungen mit einer Partikelgröße von 0,5-500 µm vorhanden sind.

2. Knochenzementpaste nach Anspruch 1, **dadurch gekennzeichnet**, wobei als Komponente **AV** ferromagnetische Partikel vorhanden sind.

3. Knochenzementpaste nach mindestens einem der Ansprüche 1 oder 2, wobei Komponente **AI** destillierbar ist.

4. Knochenzementpaste nach mindestens einem der vorstehenden Ansprüche, wobei Komponente **AI** einen Siedepunkt über 110 °C besitzt.

5. Knochenzementpaste nach mindestens einem der vorstehenden Ansprüche, wobei ein Gewichtsverhältnis von 25-50 Gewichtsteilen Methacrylatmonomer/Methacrylatmonomeren zu 2-35 Gewichtsteilen lösliche Polymere zu 40-70 Gewichtsteilen im Methylmethacrylatmonomer/Methylmethacrylatmonomeren unlösliches Polymer vorliegt.

6. Knochenzementpaste nach mindestens einem der vorstehenden Ansprüche, **dadurch gekennzeichnet, dass** das Polymer **AII** den Poly-methylmethacrylat-copolymeren angehört.

7. Knochenzementpaste nach Anspruch 6, **dadurch gekennzeichnet, dass** das Polymer **AII** Poly-methylmethacrylat-co-methylacrylat oder Poly-methylmethacrylat-co-styren ist.

8. Knochenzementpaste nach Anspruch 7, **dadurch gekennzeichnet, dass** das Polymer **AIII** vernetztes Poly-methylmethacrylat oder vernetztes Poly-methylmethacrylat-co-methylacrylat ist.

9. Knochenzementpaste nach mindestens einem der vorstehenden Ansprüche, **dadurch gekennzeichnet, dass** als Methacrylatmonomer mindestens ein difunktionelles Methacrylat enthalten ist.

10. Knochenzementpaste nach Anspruch 9, **dadurch gekennzeichnet, dass** als Methacrylatmonomer mindestens ein Mitglied der Gruppe bestehend aus Ethylenglykoldimethacrylat, Butan-1,3-diol-dimethacrylat, Butan-1,4-diol-dimethacrylat und Hexan-1,6-diol-dimethacrylat enthalten ist.

11. Knochenzementpaste nach einem der vorstehenden Anspruch, **dadurch gekennzeichnet, dass** als Komponente **AIV** mindestens ein Barbitursäurederivat enthalten ist.

12. Knochenzementpaste nach Anspruch 11, **dadurch gekennzeichnet, dass** mindestens ein Akzelerator enthalten ist.

13. Knochenzementpaste nach Anspruch 12, **dadurch gekennzeichnet, dass** als Akzelerator mindestens ein organisches Kupfer(II)-salz enthalten ist.

14. Knochenzementpaste nach einem der vorangegangenen Ansprüche, **dadurch gekennzeichnet, dass** mindestens ein weiterer Röntgenopaker enthalten ist

15. Knochenzementpaste nach Anspruch 14, **dadurch gekennzeichnet, dass** der Röntgenopaker der Gruppe bestehend aus Zirkoniumdioxid, Bariumsulfat, Tantal und biokompatiblen Calciumsalzen entstammt.

16. Knochenzementpaste nach einem der vorstehenden Ansprüche, **dadurch gekennzeichnet, dass** mindestens ein pharmazeutischer Wirkstoff aus den Gruppen der Antibiotika, Hormone, Wachstumsfaktoren und Antiphlogistika enthalten ist.

17. Knochenzementpaste nach einem der vorstehenden Ansprüche, **dadurch gekennzeichnet, dass** bis zu 20 Gewichtsteile mindestens eines biokompatiblen partikulären oder im Methacrylatmonomer/Methacrylatmonomeren löslichen Elastomers enthalten sind.

18. Knochenzementpaste nach Anspruch 17, **dadurch gekennzeichnet, dass** Polybutadien und/oder Poly-butadien-co-styrol enthalten ist/sind.

19. Verwendung einer Knochenzementpaste nach mindestens einem der Ansprüche 1-18 zur Herstellung eines Mittels zur Fixierung von Totalendoprothesen und Revisionsendoprothesen.

20. Verwendung einer Knochenzementpaste nach mindestens einem der Ansprüche 1-18 zur Herstellung eines selbsthärtenden Füllmaterials für die Vertebroplastie, Kyphoplastie oder Femurhalsaugmentation.

21. Knochenzementpaste nach mindestens einem der Ansprüche 1-18 zur Verwendung bei der Herstellung von lokalen Wirkstofffreisetzungssystemen.

22. Knochenzementpaste nach einem der Ansprüche 1-18, zur Verwendung in einem Verfahren zur Aushärtung der Knochenzementpaste, bei dem die Knochenzementpaste einem magnetischen Wechselfeld mit einer Frequenz im Bereich von 500 Hz bis 50 kHz ausgesetzt wird.

23. Knochenzementpaste nach mindestens einem der Ansprüche 2 - 18, zur Verwendung in einem Verfahren zur Aushärtung der Knochenzementpaste, bei dem die Knochenzementpaste durch Induktion bis zum Einsetzen des Zerfalls des Initiators AV erwärmt wird.

## Claims

1. One-component PMMA bone cement paste containing
**AI.** at least one methacrylate monomer for radical polymerisation;
**AII**. at least one polymer that is soluble in **AI;**
**AIII.** at least one particulate polymer that is not soluble in **AI** and has a particle size of less than 500 µm;
**AIV.** at least one thermally-decomposing radical initiator that is soluble in **AI;**
**AV.** particles of cobalt, iron, NdFeB, SmCo, cobalt-chromium steel, zirconium, hafnium, titanium, titanium-aluminium-silicon alloys, and titanium-niobium alloys with a particle size of 0.5-500 µm.

2. Bone cement paste according to claim 1, **characterised in that** ferromagnetic particles are present as component **AV.**

3. Bone cement paste according to at least one of the claims 1 or 2, whereby component **AI** can be distilled.

4. Bone cement paste according to at least one of the preceding claims, whereby component **AI** has a boiling point above 110°C.

5. Bone cement paste according to at least one of the preceding claims, whereby a weight ratio of 25-50 parts by weight methacrylate monomer/methacrylate monomers to 2-35 parts by weight soluble polymers to 40-70 parts by weight polymer that is insoluble in the methylmethacrylate monomer/methylmethacrylate monomers is established.

6. Bone cement paste according to at least one of the preceding claims, **characterised in that** polymer **AII** is a poly-methylmethacrylate-co-polymer.

7. Bone cement paste according to claim 6, **characterised in that** polymer **AII** is poly-methylmethacrylate-co-methylacrylate or poly-methylmethacrylate-co-styrene.

8. Bone cement paste according to claim 7, **characterised in that** polymer AIII is cross-linked poly-methylmethacrylate or cross-linked poly-methylmethacrylate-co-methylacrylate.

9. Bone cement paste according to at least one of the preceding claims, **characterised in that** it contains at least one di-functional methacrylate as methacrylate monomer.

10. Bone cement paste according to claim 9, **characterised in that** it contains at least one member of the group consisting of ethylene glycol dimethacrylate, butane-1,3-diol-dimethacrylate, butane-1,4-diol-dimethacrylate, and hexane-1,6-diol-dimethacrylate as methacrylate monomer.

11. Bone cement paste according to any one of the preceding claims, **characterised in that** it contains at least one barbituric acid derivative as component **AIV.**

12. Bone cement paste according to claim 11, **characterised in that** it contains at least one accelerator.

13. Bone cement paste according to claim 12, **characterised in that** it contains at least one organic copper(II) salt as accelerator.

14. Bone cement paste according to any one of the preceding claims, **characterised in that** it contains at least one further radiopaquer.

15. Bone cement paste according to claim 14, **characterised in that** the radiopaquer originates from the group consisting of zirconium dioxide, barium sulfate, tantalum, and biocompatible calcium salts.

16. Bone cement paste according to any one of the preceding claims, **characterised in that** it contains at least one pharmaceutical agent from the group of antibiotics, hormones, growth factors, and anti-phlogistic agents.

17. Bone cement paste according to any one of the preceding claims, **characterised in that** it contains up to 20 parts by weight of at least one biocompatible elastomer that is particulate or soluble in the methacrylate monomer/methacrylate monomers.

18. Bone cement paste according to claim 17, **characterised in that** it contains poly-butadiene and/or poly-butadiene-co-styrene.

19. Use of a bone cement paste according to at least one of the claims 1-18 for production of a means for fixation of total endoprostheses and revision endoprostheses.

20. Use of a bone cement paste according to at least one of the claims 1-18 for production of a self-curing filling material for vertebroplasty, kyphoplasty or femoral neck augmentation.

21. Bone cement paste according to at least one of the claims 1-18 for use in the production of local agent release systems.

22. Bone cement paste according to any one of the claims 1-18 for use in a method for curing the bone cement paste, in which the bone cement paste is exposed to an alternating magnetic field with a frequency in the range of 500 Hz to 50 kHz.

23. Bone cement paste according to at least one of the claims 2-18 for use in a method for curing the bone cement paste, in which the bone cement paste is heated by induction until the decomposition of the initiator **AV** commences.

## Revendications

1. Pâte de ciment osseux de PMMA à une composante contenant
**AI.** au moins un monomère de méthacrylate polymérisable par voie radicalaire,
**AII.** au moins un polymère soluble dans **AI,**
**AIII.** au moins un polymère particulaire non soluble dans **AI** avec une taille particulaire inférieure à 500 µm,
**AIV.** au moins un initiateur radicalaire à décomposition thermique, soluble dans **AI,**
**AV.** des particules de cobalt, fer, NdFeB, SmCo, cobalt-chrome-acier, zirconium, hafnium, titane, alliages de titane-aluminium-silicium et alliages de titane-niobium avec une taille particulaire de 0,5 à 500 µm.

2. Pâte de ciment osseux selon la revendication 1, **caractérisée en ce que** des particules ferromagnétiques sont présentes en tant que composante **AV.**

3. Pâte de ciment osseux selon au moins une des revendications 1 ou 2, dans laquelle la composante **AI** peut être distillée.

4. Pâte de ciment osseux selon au moins une des revendications précédentes, dans laquelle la composante **AI** possède un point d'ébullition au-dessus de 110°C.

5. Pâte de ciment osseux selon au moins une des revendications précédentes, dans laquelle un rapport pondéral de 25 à 50 parties en poids de monomère de méthacrylate/monomères de méthacrylate sur 2 à 35 parties en poids de polymères solubles sur 40 à 70 parties en poids de polymère insoluble dans le monomère de méthylméthacrylate/monomères de méthylméthacrylate est présent.

6. Pâte de ciment osseux selon au moins une des revendications précédentes, **caractérisée en ce que** le polymère **AII** appartient aux copolymères de polyméthylméthacrylate.

7. Pâte de ciment osseux selon la revendication 6, **caractérisée en ce que** le polymère **AII** est un poly-méthylméthacrylate-co-méthylacrylate ou polyméthylméthacrylate-co-styrène.

8. Pâte de ciment osseux selon la revendication 7, **caractérisée en ce que** le polymère **AIII** est un poly-méthylméthacrylate réticulé ou polyméthylméthacrylate-co-méthylacrylate réticulé.

9. Pâte de ciment osseux selon au moins une des revendications précédentes, **caractérisée en ce qu'**au moins un méthacrylate difonctionnel est contenu en tant que monomère de méthacrylate.

10. Pâte de ciment osseux selon la revendication 9, **caractérisée en ce qu'**au moins un membre du groupe constitué du diméthacrylate d'éthylèneglycol, butane-1,3-diol-diméthacrylate, butane-1,4-diol-diméthacrylate et hexane-1,6-diol-diméthacrylate est contenu en tant que monomère de méthacrylate.

11. Pâte de ciment osseux selon une des revendications précédentes, **caractérisée en ce qu'**au moins un dérivé d'acide barbiturique est contenu en tant que composante **AIV.**

12. Pâte de ciment osseux selon la revendication 11, **caractérisée en ce qu'**au moins un accélérateur est contenu.

13. Pâte de ciment osseux selon la revendication 12, **caractérisée en ce qu'**au moins un sel de cuivre(II) organique est contenu en tant qu'accélérateur.

14. Pâte de ciment osseux selon une des revendications précédentes, **caractérisée en ce qu'**au moins un autre opacifiant radiographique est contenu.

15. Pâte de ciment osseux selon la revendication 14, **caractérisée en ce que** l'opacifiant radiographique provient du groupe constitué du dioxyde de zirconium, sulfate de baryum, tantale et de sels de calcium biocompatibles.

16. Pâte de ciment osseux selon une des revendications précédentes, **caractérisée en ce qu'**au moins un ingrédient actif pharmaceutique provenant des groupes des antibiotiques, hormones, facteurs de croissance et anti-inflammatoires est contenu.

17. Pâte de ciment osseux selon une des revendications précédentes, **caractérisée en ce que** jusqu'à 20 parties en poids d'au moins un élastomère particulaire biocompatible ou soluble dans le monomère de méthacrylate/monomères de méthacrylate sont contenues.

18. Pâte de ciment osseux selon la revendication 17, **caractérisée en ce que** du polybutadiène et/ou poly-butadiène-co-styrène est/sont contenu(s).

19. Utilisation d'une pâte de ciment osseux selon au moins une des revendications 1 à 18 pour la fabrication d'un moyen pour la fixation d'endoprothèses totales et d'endoprothèses de révision.

20. Utilisation d'une pâte de ciment osseux selon au moins une des revendications 1 à 18 pour la fabrication d'un matériau de remplissage autopolymérisable pour la vertébroplastie, la kyphoplastie ou l'augmentation du col du fémur.

21. Pâte de ciment osseux selon au moins une des revendications 1 à 18 pour l'utilisation lors de la fabrication de systèmes de libération d'ingrédient actif locaux.

22. Pâte de ciment osseux selon une des revendications 1 à 18 pour l'utilisation dans un procédé pour le durcissement de la pâte de ciment osseux, lors duquel la pâte de ciment osseux est exposée à un champ magnétique alternatif avec une fréquence dans la région de 500 Hz à 50 kHz.

23. Pâte de ciment osseux selon au moins une des revendications 2 à 18 pour l'utilisation dans un procédé pour le durcissement de la pâte de ciment osseux, lors duquel la pâte de ciment osseux est chauffée par induction jusqu'au commencement de la décomposition de l'initiateur AV.
